# EUROPÄISCHE PATENTSCHRIFT

(11) **EP 1 048 263 B1**
(45) Veröffentlichungstag und Bekanntmachung des Hinweises auf die Patenterteilung: **16.08.2006**
(21) Anmeldenummer: 00108913.5
(22) Anmeldetag: 27.04.2000
(51) Int. Cl.: A61B 3/12, G01N 21/64

(54) **Verfahren und Anordnung zur Bestimmung von Fluorophoren an Objekten, insbesondere am lebenden Augenhintergrund**
Method and device for determining fluorophores, in particular in the living eye fundus
Méthode et dispositif de détermination de fluorophores, en particulier sur un fond d'oeil vivant

(30) Priorität: 29.04.1999 DE 19920158
(43) Veröffentlichungstag der Anmeldung: 02.11.2000
(73) Patentinhaber: Friedrich-Schiller-Universität Jena, 07743 Jena (DE)
(72) Erfinder: Schweitzer, Dietrich, Doz. Dr., 07806 Neustadt/Orla (DE); Kolb, Achim, Dipl.-Phys., 07768 Kahla (DE); Hammer, Martin, Dipl.-Phys., 07749 Jena (DE); Thamm, Eike, Dr. rer. nat., 07751 Maua (DE)
(74) Vertreter: Schmitt, Meinrad

(56) Entgegenhaltungen:
- DE-A- 19 722 790
- US-A- 4 569 354
- AMMASI PERIASAMY ET AL: "TIME-RESOLVED FLUORESCENCE LIFETIME IMAGING MICROSCOPY USING A PICOSECOND PULSED TUNABLE DYE LASER SYSTEM" REVIEW OF SCIENTIFIC INSTRUMENTS, AMERICAN INSTITUTE OF PHYSICS. NEW YORK, US, Bd. 67, Nr. 10, 1. Oktober 1996 (1996-10-01), Seiten 3722-3731, XP000635833 ISSN: 0034-6748
- BRISMAR E: "Time correlated single photon counting using a CSLM" THREE-DIMENSIONAL MICROSCOPY: IMAGE ACQUISITION AND PROCESSING, SAN JOSE, CA, USA, 7-8 FEB. 1994, Bd. 2184, Seiten 82-92, XP009020084 Proceedings of the SPIE - The International Society for Optical Engineering, 1994, USA ISSN: 0277-786X
- DOCCHIO F: "Ocular fluorometry: principles, fluorophores, instrumentation, and clinical applications." LASERS IN SURGERY AND MEDICINE. UNITED STATES 1989, Bd. 9, Nr. 6, 1989, Seiten 515-532, XP009020032 ISSN: 0196-8092

## Beschreibung

Die Erfindung betrifft ein Verfahren und eine Anordnung zur Bestimmung von Fluorophoren an Objekten, insbesondere am lebenden Augenhintergrund, gemäß der Oberbegriffe des Patentanspruches 1 sowie des Patentanspruchs 9.

Ein derartiges Verfahren und eine derartige Anordnung sind aus der DE-A-197 22 790 bekannt, wobei zur Bestimmung von Fluorophoren an Objekten, insbesondere am lebenden Augenhintergrund, durch pulsförmiges Laserlicht das Objekt punktförmig beleuchtet sowie zur Fluoreszenz angeregt und das entstehende Fluoreszenzlicht zur Auswertung abgetastet wird. Das Fluoreszenzlicht wird in einer indirekten zeitkorrelierten Einzelphotonenzählung durch einen ein- oder zweidimensionalen Akumulierungsvorgang detektiert und aus dem derart bestimmten zeitlichen Fluoreszenzverhalten werden die Fluoreszenzabklingzeiten bestimmt. Auftretendes Reflektionslicht kann zu nicht unerheblichen Verfälschungen der Messergebnisse führen.

Ferner ist aus der Zeitschrift "REVIEW OF SCIENTIFIC INSTRUMENTS, AMERICAN INSTITUTE OF PHYSICS. NEW YORK, US, Bd. 67, Nr. 10, 1. Oktober 1996 (1996-10-01), Seiten 3722-3731, AMMASI PERIASAMY ET AL: TIME-RESOLVED FLUORESCENCE LIFETIME IMAGING MICROSCOPY USING A PICOSECOND PULSED TUNABLE DYE LASER SYSTEM", die Messung der Fluoreszenzabklingzeit mittels direkter zeitkorellierter Photonenzählung bekannt. Des Weiteren ist in der Zeitschrift "Proceedings of the SPIE - The International Society for Optical Engineering, 1994, USA, THREE-DIMENSIONAL MICROSCOPY: IMAGE ACQUISITI-ON AND PROCESSING, SAN JOSE, CA, USA, 7-8 FEB. 1994, Bd. 2184, Seiten 82-92, BRISMAR E: Time correlated single photon counting using a CSLM" die zeitkorellierte Einzelphotonenzählung und die Bestimmung von Abklingzeiten beschrieben.

Es ist bekannt, zur Analyse von Objekten, beispielsweise zur Bewertung des Stoffwechselzustandes von lebendem biologischen Gewebe, wie des Augenhintergrundes bei ophthalmologischen Untersuchungen, die natürliche Fluoreszenz der Objekte auszunutzen. Durch Fluoreszenzanregung dieser Objekte kann somit nichtinvasiv durch optische Auswertung der angeregten Autofluoreszenzen in biologischem Gewebe auf dessen Stoffwechselzustand geschlossen werden. Erforderlich ist eine mindestens zweidimensionale Angabe der Verteilung verschiedener Fluorophore im untersuchten biologischen Objekt.

Die Untersuchung des Gefäßsystems des Augenhintergrundes bei ophthalmologischen Untersuchungen mit Hilfe von Fluoreszenzmarkern, z. B. unter Verwendung von intravenös injiziertem Natrium-Fluoreszein (Quantenwirkungsgrad ca. 1) oder Indocyaningrün ist Stand in der klinischen Routine. Die Messung der Autofluoreszenz am Augenhintergrund ist nur mit einem sehr geringen Quantenwirkungsgrad möglich, wobei zusätzlich geringe Konzentrationen der Fluorophore vorliegen. Erschwerend kommt hinzu, dass zur Anregung der Autofluoreszenz die Grenzwerte für die maximal zulässige Lichtbelastung (American National Standard for Use of Lasers, ANSI 136. 1-1993) eingehalten werden müssen. In der US 4 569 345 wird vorgeschlagen, die Oxygenation der Retina durch die Messung der Autofluoreszenz bei 520 nm und 540 nm zu beurteilen, wobei die Anregung bei 450 nm erfolgt. Nachteilig ist, daß die Anregung bei 450 nm zu einer Absorption in der Augenlinse führt, wodurch die Linse zur Fluoreszenz angeregt wird. Aus den Messungen der Fluoreszenz bei 520 nm und bei 540 nm soll der Einfluß der Okularmedien, wie Linse, Kammerwasser, Hornhaut und Glaskörper, kompensiert werden. Da die Fluoreszenz der Augenlinse um ca. drei Größenordnungen intensiver als die Fluoreszenz am Augenhintergrund ist, ist es kaum möglich, auswertbare Fluoreszenzsignale vom Augenhintergrund in schmalen Wellenlängenbereichen zu erreichen und diese zur Kompensation des Einflusses der Okularmedien zu verwenden. Durch die Bestrahlung bei 450 nm werden auch Produkte der Lipidperoxidation zur Fluoreszenz angeregt, die ebenfalls eine meßbare Fluoreszenz bei 540 nm zeigen. Das bedeutet, daß die Einflüsse von Linsenfluoreszenz, von Flavoproteinen des Augenhintergrundes und von Produkten der Lipidperoxidation am Augenhintergrund selbst bei bekannten Fluoreszenzspektren der einzelnen Fluorophore nicht durch die Messung der Fluoreszenz bei zwei Wellenlängen getrennt bestimmt werden können.

In der US 4 213 678 ist das Prinzip zur konfokalen Abtastung des Augenhintergrundes mit einem Laser-Scanner-Ophthalmoskop angegeben. Nach diesem Prinzip werden Reflexionsbilder des Augenhintergrundes erhalten, nachdem ein kontinuierlich leuchtender Laserstrahl scannend den Augenhintergrund bestrahlt und das Reflexionslicht von einem Detektor registriert wird. Unter Verwendung von fluoreszierenden Markern (Natrium-Fluoreszein, ICG) kann mit dieser Technik eine Beurteilung des retinalen oder chorioidalen Gefäßsystems erfolgen. Es wurde weiterhin von v. Rückmann et al. (Br. J. Ophthalmol 1995; 79: 407-412) gezeigt, daß insbesondere bei altersentsprechender Makuladegeneration (AMD) eine Autofluoreszenz nachweisbar ist, wenn der Augenhintergrund mit Licht der Wellenlänge 488 nm angeregt und das gesamte Fluoreszenzlicht global oberhalb einer cut off Wellenlänge von 520 nm integral registriert wird. Durch die Überlagerung mehrerer Autofluoreszenzbilder wird das Signal-RauschVerhältnis verbessert.

Zur Charakterisierung vorwiegend des pathologischen Zustandes durch die Spektren der Autofluoreszenz spezifischer Fluorophore bei z. B. altersbedingter Makuladegeneration (AMD) wurden verschiedene Prinzipien publiziert. Durch die Anregung eines verhältnismäßig großen Bereiches am. Augenhintergrund und die Messung des Spektrums der Autofluoreszenz konnten Delori et al. (Invest Ophthalmol Vis Sci. 1995; 36: 718 - 729) zeigen, daß das Abbauprodukt der Lipidperoxidation, Lipofuszin, zu einem großen Teil die Autofluoreszenz bei AMD bestimmt.

Durch die Anwendung der Imaging Spektrometrie, bei der nach Anregung der Autofluoreszenz längs einer Linie simultan die Spektren der Autofluoreszenz aller angeregten Orte des Augenhintergrundes gemessen werden, wurde durch Schweitzer et al. (Invest Ophthalmol Vis Sci. 1998; 38: 387) nach Anregung in verschiedenen Wellenlängenbereichen gezeigt, daß mindestens für zwei Fluorophore am Augenbintergrund eine Autofluoreszenz anregbar ist. Während die kürzerwellige Autofluoreszenz bei Angengesunden stärker ausgeprägt ist, ergibt sich bei AMD ein langwellig stärker ausgeprägtes Fluoreszenzspektrum. Das biologische Gewebe, speziell des Augenhintergrundes, besitzt die Eigenschaft, daß durch jede mögliche Bestrahlung im sichtbaren Spektralbereich mehrere Fluorophore angeregt werden, deren Fluoreszenzspektren einander überdecken. Dabei ist der Quantenwirkungsgrad der natürlichen Fluorophore sehr klein. Da der Augenhintergrund die lichtempfindlichen Rezeptoren des Auges enthält, muß die Anregungsintensität so klein sein, daß jegliche Schädigung ausgeschlossen ist. Für eine klinische Anwendung der Autofluoreszenz zur Charakterisierung des Stoffwechselzustandes oder dessen Veränderung zu einem Zeitpunkt, bevor morphologische Veränderungen erkannt werden, ist des weiteren eine zweidimensionale Messung der Autofluoreszenz mit einer möglichst hohen Ortsauflösung erforderlich, die ebenfalls zu einer Verringerung des Fluoreszenzlichtes führt, das von jedem Ort nachweisbar ist. Je feiner die Ortsauflösung pro Meßpunkt ist, desto geringer ist die meßbare Fluoreszenzintensität. Besonders erschwerend für die Messung der Autofluoreszenz am Augenhintergrund ist die Tatsache, daß das Auge ein bewegliches Objekt ist, dem es nur eine begrenzte Zeit gelingt, ein Objekt zu fixieren. Als Konsequenz aus diesen besonderen Untersuchungsbedingungen ergibt sich, daß eine Trennung der am Augenhintergrund wirksamen Fluorophore durch die unterschiedlichen Fluoreszenzspektren infolge der überlappenden Fluoreszenzspektren und des schlechten Signal-Rausch-Verhältnisses, mit dem die Autofluoreszenz am Augenhintergrund meßbar ist, aus gegenwärtiger Sicht praktisch nicht realisiert werden kann.

Es besteht jedoch in der Fachwelt ein großes Interesse, gerade am Augenhintergrund die Autofluoreszenz unterschiedlicher Fluorophore zur Kennzeichnung des Stoffwechselstatus zweidimensional zu erfassen, was mit den bekannten Methoden und unter den besagten besonderen ophthalmologischen Bedingungen effektiv nicht möglich ist.

Der Erfindung liegt deshalb die Aufgabe zu Grunde, sich in Anregungs- und/oder Fluoreszenzspektren zumindest teilweise überlappende Fluorophore von Objekten auch bei sehr geringen Fluoreszenzintensitäten sicher zu unterscheiden und für eine Auswertung mit der Möglichkeit einer zweidimensionalen Darstellung zu selektieren.

Erfindungsgemäß werden ein Verfahren und eine Anordnung vorgeschlagen, mit dem bzw. der das Objekt, beispielsweise der Augenhintergund für ophthalmologische Untersuchungen, mit gepulstem Laserlicht punktförmig beleuchtet und mit zweidimensionaler Ausdehnung zur Autofluoreszenz angeregt wird. Das nach Anregung durch jeden Laserimpuls kurzzeitig entstehende Fluoreszenzlicht wird in zeitkorrelierter Einzelphotonenzählung detektiert. Aus dem durch die zeitkorrelierte Einzelphotonenzählung bestimmten zeitlichen Fluoreszenzverhalten für jeden Ort werden die Fluoreszenz-Zeitkonstanten berechnet. Da die Fluoreszenz-Zeitkonstante ein charakteristisches Merkmal für jedes Fluorophor ist, werden aus den Fluoreszenz-Zeitkonstanten auf die im Objekt angeregten Fluorophore geschlossen. Während nach dem bekannten Stand der Technik die Unterscheidung verschiedener Fluorophore, insbesondere des Augenhintergrundes, in einer ortsaufgelösten zweidimensionalen Darstellung auf Grund der überlappenden Fluoreszenzspektren und der extrem geringen Fluoreszenzintensität als Folge der Begrenzung der Anregungsstrahlungsleistung entsprechend den Sicherheitsvorschriften nicht erreicht werden kann, ist eine Unterscheidung der Fluorophore entsprechend ihrer unterschiedlicher Fluoreszenzahklingzeit möglich. Überraschenderweise ergab sich, daß die äußerst schwachen Fluoreszenzintensitäten, die vom Augenhintergrund meßbar sind, sehr gute Bedingungen für ein zeitkorreliertes Einzelphotonenzählen darstellen. Die Technik des zeitkorrelierten Einzelphotonenzählens erfordert, daß durch einen Laser-Anregungsimpuls nur mit einer Wahrscheinlichkeit von 0,1 ein Fluoreszenzphoton registriert wird. Die Folge der Anregungsimpulse muß hoch sein, damit ein auswertbares Abklingverhalten in einer entsprechend kurzen Zeit registriert werden kann. Das Zeitregime für die zeitkorrelierte Einzelphotonenzählung wird durch die detektierten Impulse des fluoreszenzanregenden Laserlichtes sowie des am Objekt entstehenden Fluoreszenzlichtes gesteuert. Aus den Registerinhalten von Zählern, in die das jeweils nach einem Anregungsimpuls detektierte Fluoreszenzphoton entsprechend der Verzögerungszeit zwischen Anregungsimpuls und diesem eingelesen wird, werden für jeden Ort des Objektes die Abklingzeiten der durch die Impulsanregung kurzzeitigen Autofluoreszenz ermittelt, nach denen die Fluorophore unterschieden werden. In den Unteransprüchen 2-8 und 10-15 sind vorteilhafte Ausführungen des im Hauptanspruch 1 genannten Verfahrens bzw. der Anordnung gemäß Hauptanspruch 9 aufgeführt.

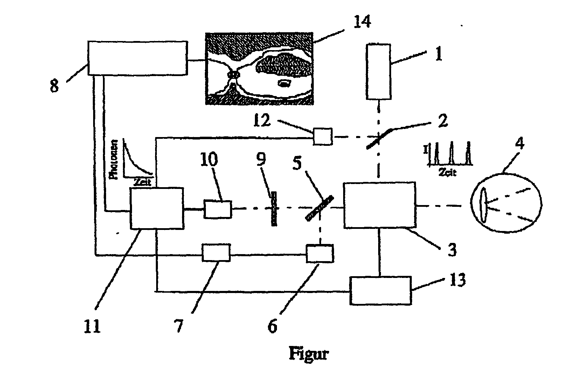

Die Erfindung soll nachstehend anhand eines in der Zeichnung dargestellten Ausführungsbeispiels näher erläutert werden.

Die Figur zeigt den Prinzipaufbau der erfindungsgemäßen Anordnung zur Auswertung von Fluorophoren an einem Augenhintergrund.

Die Strahlung eines gepulsten Lasers 1 mit einer Pulslänge von kleiner 0,5 ns und einer Folgefrequenz von ca. 80 MHz wird über einen teildurchlässigen Spiegel 2 in ein Laser-Scanner-Ophthalmoskop 3 eingekoppelt und beleuchtet den Hintergrund eines zu untersuchenden Patientenauges 4 punktweise durch Ablenkung des Laserstrahlimpulses in horizontaler und vertikaler Richtung. Zur Diskriminierung von Reflexlicht von den vorderen Augenmedien und zur Unterdrückung von Fluoreszenzlicht, das in den vorderen Augenmedien, insbesondere in der Augenlinse, angeregt wird, erfolgt eine geometrische Trennung von Beleuchtungsstrahlengang und Detektionsstrahlengang durch Aperturblendenteilung in der Aperturblendenebene des Patientenauges 4 (aus Übersichtsgründen nicht in der Zeichnung dargestellt). Das vom Augenhintergrund des Patientenauges 4 reflektierte Licht erfährt beim erneuten Durchlauf durch das Laser Scanner Ophthalmoskop 3 ein Descannen und wird nach Ablenkung durch einen dichroitischen Spiegel 5 in bekannter Weise von einem Detektor 6 registriert, auf den der beleuchtete Ort des Augenhintergrundes konfokal abgebildet wurde. Über einen mit dem Laser-Scanner-Ophthalmoskop 3 synchronisierten Framegrabber 7 werden die Reflexionsbilder des Augenhintergrundes in einen Rechner 8 eingelesen.

Die Strahlung des gepulsten Lasers 1 hat eine wählbare Wellenlänge, die im vorliegenden Beispiel kurzwelliger als 520 nm gewählt wird. Mit dieser Strahlung werden Fluorophore des Augenhintergrundes zu einer kurzzeitigen Fluoreszenz angeregt. Das Fluoreszenzlicht erfährt ebenfalls durch das Laser-Scanner-Ophthalmoskop 3 ein Deseannen und trifft auf den dichroitischen Spiegel 5, dessen Eigenschaften so gewählt sind, daß Licht im Wellenlängenbereich größer als 520 nm durch den Spiegel 5 transmittiert, während das kurzwelligere Anregungslicht für die Fluoreszenz am Spiegel 5 reflektiert wird. Die Trennung von Fluoreszenzlicht und Reflexionslicht am dichroitischen Spiegel 5 kann auch so gewählt werden, daß das Fluoreszenzlicht an diesem reflektiert wird, während das vom Patientenauge 4 reflektierte Licht durch diesen transmittiert. Das Fluoreszenzlicht tritt anschließend durch einen Sperrfilter 9, der dasjenige Anregungslicht, welches durch den dichroitischen Spiegel 5 ungenügend gesperrt sein sollte, sowie ggf. geräteinternes Streulicht vollständig blockt. Das Fluoreszenzlicht wird von einem hochempfindlichen Fluoreszenzdetektor 10 registriert, der an eine Einheit 11 zur zeitkouelierten Einzelphotonenzählung angeschlossen ist.

Beim zeitkorrelierten Einzelphotonenzählen wird im Normalbetrieb durch jeden Anregungsimpuls ein Zeitvorgang gestartet, der durch das registrierte einzelne Fluoreszenzphoton gestoppt wird. Der gesamte Zeitvorgang ist in Abschnitte unterteilt, denen entsprechend der gemessenen Verzögerungszeit zwischen Anregungsimpuls und Fluoreszenzphoton einzelne Register zugeordnet sind, die nacheinander für jedes registrierte Fluoreszenzphoton um eins höhergezählt werden. Nachdem eine Folge von Anregungsimpulsen auf den Augenbintergrund getroffen ist, repräsentieren die Inhalte der einzelnen Zähler der Einheit 11 das zeitliche Abklingverhalten des pulsförmig angeregten Fluoreszenzlichtes an einem Ort. Im Rechner 8 werden aus dem zeitlichen Abklingverhalten der Fluoreszenz die Zeitkonstanten (decay time) des zeitlichen Fluoreszenzverhaltens bestimmt. Anhand dieser Zeitkonstanten der Fluoreszenz werden Fluorophore und deren Konzentration unterschieden. Ein besonderer Vorteil dieser erfindungsgemäßen Unterscheidung der Fluorophore nach dem Abklingverhalten des Fluoreszenzlichtes besteht darin, daß die Auswertung unabhängig von der Fluoreszenzintensität und damit auch für beispielsweise in der Ophthalmologie auftretende sehr geringe Fluoreszenzintensitäten anwendbar ist. Die auf diese Weise im Fluoreszenzbild des Augenhintergrundes des Patientenauges 4 unterscheidungsfähigen Fluorophore werden im Rechner 8 aus den bekannten Zuordnungen zwischen Abklingzeitkonstante und Fluorophor bei bekannter Anregungswellenlänge bestimmt.

Um die vom Augenhintergrund registrierte Fluoreszenz entsprechend dem zeitkorrelierten Einzelphotonenzählen nachweisen zu können, erfolgt eine Zeitsynchronisation zwischen dem gepulsten Lasers 1 und der Einheit 11 zur zeitkorrelierten Einzelphotonenzählung. Dazu wird ein Teil des Laser-Anregungspulses durch den teildurchlässigen Spiegel 2 ausgespiegelt und von einem schnellen Detektor 12 (Anregungsdetektor) registriert Dessen Ausgangssignal startet den Zeitprozeß der Einheit 11 zur zeitkorrelierten Einzelphotonenzählung. Unter Beachtung der maximal zulässigen Exposition, der Transmission der optischen Anordnung, sowie des Quantenwirkungsgrades für die Anregung von Fluoreszenzlicht am Augenhintergrund und des Detektorquantenwirkungsgrades stoppt das mit einer Wahrscheinlichkeit von ca. 0,1 entstehende Ausgangssignal des Fluoreszenzdetektors 10 den Zeitprozeß der Einheit 11 zur zeitkorrelierten Einzelphotonenzählung. Die funktionelle Wirkung von Anregungsimpuls und detektiertem Fluoreszenzphoton für die Erzeugung von Start und Stopp Signal im Zeitprozeß kann prinzipiell auch umgekehrt vorgenommen werden (reversed mode), so daß das Fluoreszenzphoton den Zeitprozeß startet und der nächste Laserimpuls zur Fluoreszenzanregung des Augenhintergrundes den Zeitprozeß beendet.

Die Fluoreszenz am Patientenauge 4 wird bei stetigem Abscannen des Augenhintergnmdes gleichzeitig mit einer hohen Folge von Laserimpulsen (im vorliegenden Beispiel eine Pulsfrequenz von 80 MHz) angeregt. Da das Autofluoreszenzsignal sehr schwach ist, muß eine Integration über einen gewissen Zeitbereich vorgenommen werden, was infolge der Scanbewegung des Anregungsstrahles dazu führt, daß ein bestimmter Bereich am Augenhintergrund überstrichen wird, um eine zeitaufgelöste Fluoreszenz zu erhalten. Die Konsequenz ist, daß die örtliche Auflösung für die zweidimensionale Darstellung der zeitaufgelösten Fluoreszenz mit ca. 50 µm etwas geringer ist als im parallel registrierten Reflexionsbild. Um eine Zuordnung der gemessenen Fluoreszenzabklingkurven zum jeweiligen Abtastort der zweidimensionalen Abtastung zu gewährleisten, ist der Scann- bzw. Descannvorgang im Laser-Scanner-Ophthalmoskop 3 über einen Router 13 mit der Einheit 11 zur zeitkorrelierten Einzelphotonenzählung synchronisiert. Mit dem Router 13 wird durch die Größe eines Zeitfensters die örtliche Auflösung festgelegt, mit der eine Zuordnung der gemessenen Fluoreszenzphotonen zu der Fluoreszenzabklingkurve eines Punktes während des kontinuierlichen Überstreichens des Augenhintergrundes mit dem scannenden Anregungslaserstrahl erfolgt.

In der Einheit 11 zur zeitkorrelierten Einzelphotonenzählung wird für jeden abgetasteten Bildpunkt des Augenhintergrundes die Abklingkurve der Autofluoreszenz als Registerinhalt gespeichert und steht somit zur Bestimmung der Fluoreszenzabklingzeit für jeden Bildpunkt, die im Rechners 8 durchgeführt wird, zur Verfügung. In einer zweidimensionalen Darstellung auf einem an den Rechner 8 angeschlossenen Monitor M wird das durch Grauwerte oder Falschfarben codierte Bild der berechneten Abklingzeiten, in dem vorteilhafterweise jeweils Bereiche gleicher Abklingzeit farblich markiert sein können, mit dem Reflexionsbild zur besseren klinischen Interpretation überlagert. Bei der Entflechtung der zeitlichen Verläufe der Fluoreszenz werden sowohl die Abklingzeitkonstanten, welche der Art der Fluorophore entsprechen, als auch die Vorfaktoren bestimmt, welche die Konzentrationen der Fluorophore repräsentieren. Die Informationen über die Art der Fluorophore und über deren Konzentration können in getrennten Bildern oder in einem Bild komprimiert dargestellt werden. Beispielsweise ist es möglich, die unterschiedlichen Fluorophore in ihren zugehörigen Bereichen durch Farben zu charakterisieren, deren Sättigung der Konzentration entspricht. Aus der additiven Mischung der Farben, die jeweils einer mit bestimmter Konzentration vorhandener fluoreszierender Komponente entsprechen, kann eine Mischfarbe berechnet werden, die dem Konzentrationsverhältnis der Fluorophore entspricht. Die zweidimensionale Verteilung der Fluorophore kann anstelle des Monitors 14 oder zusätzlich zu diesem auch auf einem Drucker ausgegeben werden. Für eine effektive Berechnung und Beurteilung von zweidimensionalen Bildern nach der Fluoreszenzabklingzeit werden weiterhin der zeitliche Abfall der Fluoreszenz auch bei mehreren fluoreszierenden Komponenten durch einen monoexponentiellen Abfall approximiert und die dabei berechnete effektive Abklingzeit zur Auswertung herangezogen.

Sowohl vor dem Detektor 6 für das Reflexionslicht, als auch vor dem Fluoreszenzdetektor 10 können zweckmäßigerweise in der Zeichnung nicht dargestellte veränderbare Feldblenden angeordnet sein, welche die konfokale Registrierung zum Anregungsstrahl in verschiedenen Tiefen des Augenhintergrundes für das Reflexions- bzw. für das Fluoreszenzlicht ermöglichen.

Durch Summation der Inhalte aller Register der Einheit 11 zur zeitkorrelierten Einzelphotonenzählung für das zeitabhängige Fluoreszenzverhalten jedes abgetasteten Punktes am Augenhintergrund kann ein Bild der integralen Fluoreszenzintensität angegeben werden.

### Aufstellung der verwendeten Bezugszeichen

- 1 -: gepulster Laser
- 2 -: teildurchlässiger Spiegel
- 3 -: Laser-Scanner-Ophthalmoskop
- 4 -: Patientenauge
- 5 -: dichroitischer Spiegel
- 6 -: Detektor für Reflexionslicht
- 7 -: Framegrabber
- 8 -: Rechner
- 9 -: Sperrfilter
- 10 -: Fluoreszenzdetektor
- 11 -: Einheit zur zeitkorrelierten Einzelphotonenzählung
- 12 -: Detektor
- 13 -: Router
- 14 -: Monitor

## Patentansprüche

1. Verfahren zur Bestimmung von Fluorophoren an Objekten, insbesondere am lebenden Augenhintergrund, bei dem das Objekt durch pulsförmiges Laserlicht punktförmig beleuchtet sowie zur Fluoreszenz angeregt und das entstehende Fluoreszenzlicht zur Auswertung abgetastet wird, wobei das nach pulsförmiger Anregung entstehende Fluoreszenzlicht in zeitkorrelierter Einzelphotonenzählung detektiert wird und aus dem durch die zeitkorrelierte Einzelphotonenzählung bestimmten zeitlichen Fluoreszenzverhalten die Fluoreszenzabklingzeiten bestimmt werden und wobei aus den ermittelten Fluoreszenzabklingzeiten auf die im Objekt zur Fluoreszenz angeregten Fluorophore geschlossen wird,
**dadurch gekennzeichnet, dass** zusätzlich zu der unter zeitkorrelierter Einzelphotonenzählung erfolgenden Detektion des pulsförmig angeregten Fluoreszenzlichtes vom Objekt gleichzeitig das Reflexionslicht des Objektes ausgewertet und aus diesem ein Reflexionsbild bestimmt wird, welches dem unter zeitkorrelierter Einzelphotonenzählung erhaltenen Fluoreszenzbild des Objektes überlagert bzw. gegenübergestellt wird.

2. Verfahren nach Anspruch 1, **dadurch gekennzeichnet, dass** das Objekt durch Laserimpulse mit einer Impulsdauer möglichst kürzer als 0,5 ns, mit einer Folgefrequenz von ca. 80 MHz und mit einer Wellenlänge kleiner als 520 nm beleuchtet und zur Fluoreszenz angeregt wird.

3. Verfahren nach Anspruch 1, **dadurch gekennzeichnet, dass** die Verteilung der Fluoreszenzabklingzeiten und/oder die nach ihrer Fluoreszenzabklingzeit bestimmten Fluorophore zweidimensional in einem Fluoreszenzbild des Objektes dargestellt werden.

4. Verfahren nach Anspruch 3, **dadurch gekennzeichnet, dass** die zu den zu unterscheidenden Fluorophoren zugehörigen Bereiche im Fluoreszenzbild des Objektes jeweils verschiedenfarbig dargestellt sind.

5. Verfahren nach Anspruch 1, **dadurch gekennzeichnet, dass** die zeitkorrelierte Einzelphotonenzählung jeweils durch die detektierten Impulse des Laserlichtes gestartet und durch die detektierten Impulse des Fluoreszenzlichtes gestoppt wird.

6. Verfahren nach Anspruch 1, **dadurch gekennzeichnet, dass** die zeitkorrelierte Einzelphotonenzählung jeweils durch die detektierten Impulse des Fluoreszenzlichtes gestartet und durch die detektierten Impulse des Laserlichtes gestoppt wird.

7. Verfahren nach Anspruch 1, **dadurch gekennzeichnet, dass** zur unabhängigen Detektion von Fluoreszenz- und Reflexionslicht des Objektes beide Lichtanteile von einander getrennt werden und dass weiterhin das Fluoreszenzlicht für den Spektralbereich des Reflexionslichtes und gegebenenfalls von Streulicht geblockt wird.

8. Verfahren nach Anspruch 1, **dadurch gekennzeichnet, dass** die örtliche Zuordnung der zeitkorrelierten Einzelphotonenzählung mit der zweidimensionalen Fluoreszenzanregung des Objektes synchronisiert wird.

9. Anordnung zur Bestimmung von Fluorophoren an Objekten, insbesondere am lebenden Augenhintergrund, bestehend aus einem gepulsten Laser (1) und einem Laser-Scanner-System zur punktförmigen Beleuchtung und zweidimensionalen Fluoreszenzanregung des Objektes (42) sowie zum Descannen des durch die Fluoreszenzanregung am Objekt entstehenden Fluoreszenzlichtes, wobei der Ausgang des Laser-Scanner-Systems (3), an dem das ortsaufgelöst erfasste pulsförmig angeregte Fluoreszenzlicht des Objektes (4) austritt, mit einem Fluoreszenzdetektor (10) in Verbindung steht, wobei eine zeitkorrelierte Einzelphotonenzählung erfolgt und wobei ein Rechner (8) mit einer Ausgabeeinheit (14) zur Berechnung der jeweiligen Fluoreszenzabklingzeiten des pulsförmig angeregten Fluoreszenzlichtes vorgesehen ist,
**dadurch gekennzeichnet, dass** für die zeitkorrelierte Einzelphotonenzählung eine Einheit (11) mit dem Fluoreszenzdetektor (10) in Verbindung steht und ferner an den Rechner (8) angeschlossen ist und dass im Strahlengang des von einem dichroitischen Spiegel (5) ausgeblendeten Reflexionslichtes des Objektes (4) ein Detektor (6) für das reflektierte Licht angeordnet ist, der zur Erfassung eines Reflexionsbildes vom Objekt (4) über einen Framegrabber (7) ebenfalls mit dem Rechner (8) in Verbindung steht.

10. Anordnung nach Anspruch 9, **dadurch gekennzeichnet, dass** der gepulste Laser (1) über strahlführende Mittel (2) und einen Detektor (12) mit einem Zeitsteuereingang der Einheit (11) zurzeitkorrelierten Einzelphotonenzählung in Verbindung steht.

11. Anordnung nach Anspruch 9, **dadurch gekennzeichnet, dass** der Ausgang des Laser-Scanner-Systems (3), an dem das ortsaufgelöst erfasste, pulsförmig angeregte Fluoreszenzlicht des Objektes (4) austritt, über den dichroitischen Spiegel (5) mit dem an die Einheit (11) zur zeitkorrelierten Einzelphotonenzählung angeschlossenen Fluoreszenzdetektor (10) in Verbindung steht.

12. Anordnung nach Anspruch 11, **dadurch gekennzeichnet, das** dem Fluzoreszenzdetektor (10) ein Sperrfilter (9) zur Blockung des Spektralbereiches des Reflexionslichtes vom Objekt (4) und von eventuell auftretendem Streulicht vorgeschaltet ist.

13. Anordnung nach Anspruch 9, **dadurch gekennzeichnet, dass** das Laser-Scanner-System (3) zum Zweck einer Synchronisation des zeitabhängigen Zählvorgangs der Fluoreszenzphotonen mit der örtlich zweidimensionalen Fluoreszenzanregung des Objektes (4) über eine Router (13) mit einem Steuereingang der Einheit (11) zur zeitkorrelierten Einzelphotonenzählung gekoppelt ist.

14. Anordnung nach einem der Ansprüche 9 bis 13, **dadurch gekennzeichnet, dass** ein visuelles und archivierendes Ausgabegerät, beispielsweise ein Monitor (14), zur Ausgabe der erfassten Fluoreszenz- und Reflexionsbilder vorgesehen ist.

15. Anordnung nach einem der Ansprüche 9 bis 14, **dadurch gekennzeichnet, dass** vor dem Fluoreszenzdetektor (10) und/oder dem Detektor (6) für reflektiertes Licht veränderbare Feldblenden zur Detektion des Fluoreszenz- und/oder des Reflexionslichtes aus unterschiedlichen Ebenen des Augenhintergrundes angeordnet ist.

## Claims

1. A method for determining fluorophores in objects, in particular in the living eye fundus, in which the object is illuminated by pulsed laser light in a point-by-point manner and is excited into fluorescence, and the fluorescent light created is sampled for evaluation, wherein
the fluorescent light created by pulsed excitation is detected by time-correlated single photon counting, and the fluorescence decay times are determined from the fluorescence behaviour with time determined from the time-correlated single photon counting, and wherein
conclusions are drawn concerning the fluorophore excited into fluorescence in the object from the fluorescence decay times determined,
**characterised in that** in addition to the detection effected by time-correlated single photon counting of the fluorescence light from the object generated by pulsed excitation, the reflection light of the object is evaluated at the same time, and from this a reflection image is determined, which is overlaid or compared with the fluorescence image of the object obtained from time-correlated single photon counting.

2. The method according to Claim 1,
**characterised in that** the object is illuminated by means of laser pulses with a pulse duration preferably shorter than 0.5 ns, with a repetition frequency of approx. 80 MHz and with a wavelength shorter than 520 nm, and is excited into fluorescence.

3. The method according to Claim 1,
**characterised in that** the distribution of the fluorescence decay times and/or the fluorophore determined according to its fluorescence decay time are represented two-dimensionally in a fluorescence image of the object.

4. The method according to Claim 3,
**characterised in that** the areas relating to different fluorophores are respectively represented in the fluorescence image of the object by different colours.

5. The method according to Claim 1,
**characterised in that** the time-correlated single photon counting is started by the detected pulses of the laser light and is stopped by the detected pulses of the fluorescence light respectively.

6. The method according to Claim 1,
**characterised in that** the time-correlated single photon counting is started by the detected pulses of the fluorescence light and is stopped by the detected pulses of the laser light respectively.

7. The method according to Claim 1,
**characterised in that** for independent detection of fluorescence and reflection of light of the object the two light components are separated from each other and that furthermore the fluorescence light is blocked for the spectral range of the reflection light and, if necessary, from stray light.

8. The method according to Claim 1,
**characterised in that** the local attribution of the time-correlated single photon counting is synchronised with the two-dimensional fluorescence excitation of the object.

9. A system for determining fluorophores in objects, in particular in the living eye fundus, consisting of a pulsed laser (1) and a laser scanner system for point-by-point illumination and two-dimensional fluorescence excitation of the object (42) as well as for descanning of the fluorescence light created as a result of the fluorescence excitation on the object, wherein
the outlet of the laser scanner system (3), at which the registered spatially resolved fluorescence light from the object (4) generated by pulsed excitation exits, is connected to a fluorescence det ector (10), wherein time-correlated single photon counting takes place and wherein
a computer (8) with an output unit (14) is provided for analysis of the respective fluorescence decay times of the fluorescence light generated by pulsed excitation,
**characterised in that** for the time-correlated single photon counting a unit (11) is connected to the fluorescence detector (10) and furthermore is connected to the computer (8), and **in that** in the optical path of the reflection light of the object (4), gated by a dichroitic mirror (5), a detector (6) is positioned for the reflected light, that is similarly connected to the computer (8) for the registration of a reflection image of the object (4) via a frame grabber (7).

10. The system according to Claim 9,
**characterised in that** the pulsed laser (1) is connected via a beam guiding medium (2) and a detector (12) with a time control input of the unit (11) for the time-correlated single photon counting.

11. The system according to Claim 9,
**characterised in that** the outlet of the laser scanner system (3), at which the registered spatially resolved fluorescence light from the object (4) generated by pulsed excitation exits, is connected via the dichroitic mirror (5) with the fluorescence detector (10) connected to the unit (11) for the time-correlated single photon counting.

12. The system according to Claim 11,
**characterised in that** a blocking filter (9) is connected ahead of the fluorescence detector (10) to block the spectral range of the reflection light from the object (4) and any stray light that may appear.

13. The system according to Claim 9,
**characterised in that** for the purposes of synchronisation of the time-dependent counting process of the fluorescence photons with the local two-dimensional fluorescence excitation of the object (4) the laser scanner system (3) is coupled via a router (13) with a control input of the unit (11) for the time-correlated single photon counting.

14. The system according to one of the Claims 9 to 13,
**characterised in that** a visual and archiving output unit, for example a monitor (14), is provided for the output of the fluorescence and reflection images registered.

15. The system according to one of the Claims 9 to 14,
**characterised in that** ahead of the fluorescence detector (10) and/or the detector (6) for reflected light adjustable field stops are located for detection of the fluorescence and/or the reflection light from different planes of the eye fundus.

## Revendications

1. Procédé pour la détermination de fluorophores sur des objets, en particulier sur un fond d'oeil vivant, selon lequel l'objet est éclairé ponctuellement par une lumière laser en forme d'impulsion et est excité à la fluorescence et la lumière fluorescence en résultant est balayée pour analyse, la lumière fluorescente résultant après l'excitation par impulsion étant détectée dans un comptage de photons uniques en corrélation temporelle et les temps de déclin de fluorescence étant définis à partir du comportement de fluorescence temporel défini par le comptage de photons uniques en corrélation temporelle, les fluorophores excités à la fluorescence dans l'objet étant conclus des temps de déclin de fluorescence déterminés,
**caractérisé en ce qu'**en plus de la détection s'effectuant par comptage de photons uniques en corrélation temporelle de la lumière fluorescente excitée sous forme d'impulsion de l'objet, la lumière de réflexion de l'objet est analysée en même temps et à partir de cette dernière, il est défini une image de réflexion qui se superpose respectivement s'oppose à l'image de fluorescence obtenue par comptage de photons uniques en corrélation temporelle.

2. Procédé selon la revendication 1, **caractérisé en ce que** l'objet est éclairé par des impulsions laser avec une durée d'impulsion dans la mesure du possible plus courte que 0,5 ns avec une fréquence de répétition d'environ 80 MHz et est excité à la fluorescence à une longueur d'ondes inférieure à 520 nm.

3. Procédé selon la revendication 1, **caractérisé en ce que** la répartition des temps de déclin de fluorescence et/ou les fluorophores définis par les temps de déclin de fluorescence sont définis bidimensionnellement dans une image de fluorescence de l'objet.

4. Procédé selon la revendication 3, **caractérisé en ce que** les zones faisant partie des fluorophores à différentier sont représentées dans l'image fluorescente de l'objet respectivement avec des couleurs différentes.

5. Procédé selon la revendication 1, **caractérisé en ce que** le comptage de photons uniques en corrélation temporelle est démarré respectivement par les impulsions détectées de la lumière laser et est stoppé par les impulsions détectées de la lumière fluorescente.

6. Procédé selon la revendication 1,**caractérisé en ce que** le comptage de photons uniques en corrélation temporelle est démarré respectivement par les impulsions détectées de la lumière fluorescente et est stoppé par les impulsions détectées de la lumière laser.

7. Procédé selon la revendication 1, **caractérisé en ce que** pour la détection indépendante de la lumière fluorescente et de réflexion de l'objet, les deux parts de lumière doivent être séparées l'une de l'autre et **en ce qu'**en outre, la lumière fluorescente est bloquée pour la plage spectrale de la lumière de réflexion et éventuellement par la lumière diffusée.

8. Procédé selon la revendication 1, **caractérisé en ce que** l'attribution locale du comptage de photons uniques en corrélation temporelle est synchronisée avec l'excitation bidimensionnelle à la fluorescence de l'objet.

9. Dispositif pour la détermination de fluorophores sur des objets, en particulier sur le fond d'oeil vivant, se composant d'un laser pulsé (1) et d'un système de scanner-laser pour l'éclairage ponctuel et l'excitation bidimensionnelle à la fluorescence de l'objet (42) ainsi que pour l'action de déscannage de la lumière fluorescente se produisant par l'excitation fluorescente sur l'objet, la sortie du système de scanner-laser (3) d'où sort la lumière fluorescente de l'objet (4) excitée par impulsion et saisie par déclenchement local, est connectée au détecteur de fluorescence (10), un comptage de photons uniques en corrélation temporelle étant effectué et un ordinateur (8) étant prévu avec une unité d'édition (14) pour le calcul des temps de déclin de fluorescence respectifs de la lumière fluorescente excitée par impulsion,
**caractérisé en ce que** pour le comptage de photons uniques en corrélation temporelle, une unité (11) est en liaison avec le détecteur de fluorescence (10) et est en outre raccordée à l'ordinateur (8) et **en ce que** dans le trajet de rayon de la lumière de réflexion de l'objet (4), masquée par un miroir dichroïque (5), il est disposé un détecteur (6) pour la lumière réfléchie, lequel est relié également à l'ordinateur (8) pour la saisie d'une image de réflexion de l'objet (4) par l'intermédiaire d'un capteur de page-écran (7).

10. Dispositif selon la revendication 9, **caractérisé en ce que** pour le comptage de photons uniques en corrélation temporelle, le laser pulsé (1) est relié par des moyens de guidage de rayon (2) et un détecteur (12) à une entrée de commande temporelle de l'unité (11).

11. Dispositif selon la revendication 9, **caractérisé en ce que** la sortie du système scanner-laser (3) d'où sort la lumière fluorescente de l'objet (4) excitée par impulsion et saisie par déclenchement local, est reliée par le miroir dichroïque (5) au détecteur de fluorescence (10) raccordé à l'unité (11) pour le comptage de photons uniques en corrélation temporelle.

12. Dispositif selon la revendication 11, **caractérisé en ce qu'**il est placé avant le détecteur de fluorescence (10) un filtre de blocage (9) pour le blocage de la plage spectrale de la lumière de réflexion de l'objet (4) et éventuellement de la lumière diffusée pouvant se produire.

13. Dispositif selon la revendication 9, **caractérisé en ce que** le système laser scanner (3) est accouplé dans le but d'une synchronisation du comptage de photons uniques par dépendance temporelle avec l'excitation bidimensionnelle par fluorescence de l'objet (4) par un routeur (13) avec une entrée de commande (11) en vue du comptage de photons uniques en corrélation temporelle.

14. Dispositif selon l'une des revendications 9 à 13, **caractérisé en ce qu'**un appareil d'édition visuelle et d'archivage, par exemple un écran (14) est prévu pour l'édition des images saisies de fluorescence et de réflexion.

15. Dispositif selon l'une des revendications 9 à 14, **caractérisé en ce que** des obturateurs de champ réglables pour la détection de la lumière de fluorescence et/ou de réflexion provenant de différents niveaux du fond d'oeil sont disposés devant le détecteur de fluorescence (10) et/ou le détecteur (6) pour la lumière réfléchie.
